# EUROPEAN PATENT APPLICATION

(11) **EP 2 136 232 A1**
(43) Date of publication of application: **23.12.2009**
(21) Application number: 09162689.5
(22) Date of filing: 15.06.2009
(51) Int. Cl.: G02B 21/00, A61B 3/13, G02B 21/22

(54) **Binocular stereo video microscope apparatus**

(30) Priority: 18.06.2008 JP 2008158781
(71) Applicant: Kabushiki Kaisha TOPCON, Tokyo 174-8580 (JP)
(72) Inventor: Kitajima, Nobuaki, Tokyo 174-8580 (JP)
(74) Representative: Ilgart, Jean-Christophe

(57) **Abstract**

To provide a binocular stereo video microscope apparatus that can produce stereo images to a tester in which image forming relationships can return to a normal state without using an inverter device constituted from an optical device that inverts image forming relationships, the present invention includes observatory optical system (7, 8) that receive light beams from a tested eye (E) via an objective lens (3) and form respectively on both image receiving devices tested eye images, a monitoring device that produces each tested eye image on a same screen but sorting one tested eye image to the left and sorting another tested eye image to the right with a center set as a boundary, a front lens (6) inserted between the tested eye and the objective lens, a switch (SW2) that cancels a relationship of the tested eye images inverted in up and down as well as right and left directions.

## Description

The present invention relates to a binocular type stereo video microscope apparatus that can stereo observe an ocular fundus image on a same screen.

Conventionally, there is known a binocular stereo microscope apparatus including an illumination optical system to illuminate an interior of an eye of a patient and a pair of observatory optical systems that takes therein from two differing directions reflective beams from the interior of the eye, and configured to be capable of observing the interior of the eye of the patient in stereo.

By the way, in a stereo microscope apparatus of this kind, between a case where a front lens is inserted into an observatory light path between an objective lens and a tested eye and a case where the front lens is not inserted, an image forming relationship of the tested eye is inverted in up and down as well as right and left directions. Therefore, an inverter device as a prism unit is disposed to be able to come in an out of a middle of an observatory light path of the observatory optical system and configured to return the image forming relationship inverted in up and down as well as right and left directions to an original state.

However, because this conventional binocular stereo microscope apparatus adopts a constitution in which an inverter device constituted from an optical device is disposed in the middle of the observatory path of the observatory optical system, a whole length of a lens barrel becomes long and a distance from a tested eye to an ocular lens becomes necessarily long. In the case the conventional stereo microscope apparatus is used as the microscope apparatus for operation, a distance from an operator to a patient's eye becomes far. As a result, there is a problem that an operation becomes difficult.

In addition, because the whole length of the lens barrel becomes long, peripheral light beams projected from the interior of the eye are blocked so that it is disadvantageous that sharpness of an image of a peripheral part of a visual field lacks easily.

Furthermore, because the conventional stereo microscope apparatus has the constitution in which the optical device as the prism unit is disposed in the light path of the observatory optical system, there exist necessarily many reflective surfaces of the optical device. As a result, there is a problem that image forming performances deteriorate.

An object of the present invention is to provide a binocular stereo video microscope apparatus able to solve the above problems, in which without using an inverter device constituted from an optical device and inverting an image forming relationship, the image forming relationship can be returned to a normal state and stereo images can be presented to a tester.

In order to achieve the above object, a binocular stereo video microscope apparatus according to the present invention includes an illumination optical system that illuminates a tested eye, a right and left pair of observatory optical system that receives via an objective lens image forming light beams from a tested eye and forms an image of the tested eye respectively on both image receiving devices of a right and left pair, a monitoring device that produces each tested eye image on a same screen but sorting one tested eye image to the left and sorting another tested eye image to the right with a center set as a boundary in which the tested eye images are obtained via the right and left pair of the observatory optical system, a front lens inserted between the tested eye and the objective lens for observing an interior of the tested eye, an inverted image cancellation switch that cancels an inverted relationship of the tested eye images inverted in up and down as well as right and left directions against the tested eye in which the tested eye images are formed on each image receiving devices via the right and left pair of observatory optical system based on the insertion of the front lens between the tested eye and the objective lens, and an image processing circuit that generates a composite image of the tested eye based on pixel data obtained from both image receiving devices of the right and left pair. In the binocular stereo video microscope apparatus, the image processing circuit performs composite processing to the tested eye image formed on the left image receiving device and the tested eye image formed on the right image receiving device in which the both images keep a relationship as they are and are processed as a tested eye composite image regardless of whether the front lens is inserted or not between the tested eye and the objective lens. In addition, based on a switch state of the inverted image cancellation switch, a read out processing and scanning of the a tested eye composite image are made different between when the front lens is not inserted between the tested eye and the objective lens and when the front lens is inserted between the tested eye and the objective lens. In addition, a tested eye image based on image forming light beams to be obtained via a left observatory optical system in a left part of the same screen when the front lens is not inserted between the tested eye and the objective lens is produced as a regular image corresponding to the tested eye. In addition, a tested eye image based on image forming light beams to be obtained via a right observatory optical system in a right part of the same screen when the front lens is not inserted between the tested eye and the objective lens is produced as a regular image corresponding to the tested eye.

FIG. 1 is a schematic diagram that illustrates a constitution of an optical system of a stereo video microscope apparatus according to a first embodiment of the present invention.
FIG. 2 is a block circuit diagram that illustrates a detailed constitution of an image processing circuit illustrated in FIG. 1.
FIG. 3A is a schematic diagram of an image processing by an image processing circuit in which a front lens illustrated in FIG. 1 is not inserted between a tested eye and an objective lens, in particular, a left tested eye image formed on a left image receiving device.
FIG. 3B is a schematic diagram of an image processing by an image processing circuit in which a front lens illustrated in FIG. 1 is not inserted between a tested eye and an objective lens, in particular, a right tested eye image formed on a right image receiving device.
FIG. 3C is a schematic diagram of an image processing by an image processing circuit in which a front lens illustrated in FIG. 1 is not inserted between a tested eye and an objective lens, in particular, a schematic diagram of a writing processing of both tested eye images onto a frame memory.
FIG. 3D is a schematic diagram of an image processing by an image processing circuit in which a front lens illustrated in FIG. 1 is not inserted between a tested eye and an objective lens, in particular, a schematic diagram of a read out processing of a tested eye composite image written onto the frame memory.
FIG. 3E is a schematic diagram of an image processing by an image processing circuit in which a front lens illustrated in FIG. 1 is not inserted between a tested eye and an objective lens, in particular, a diagram that illustrates a state of display of the a tested eye composite image displayed on a monitoring device illustrated in FIG. 1.
FIG. 4A is a schematic diagram of an image processing by an image processing circuit in which a front lens illustrated in FIG. 1 is inserted between a tested eye and an objective lens, in particular, a right tested eye image formed on a left image receiving device.
FIG. 4B is a schematic diagram of an image processing by an image processing circuit in which a front lens illustrated in FIG. 1 is inserted between a tested eye and an objective lens, in particular, a left tested eye image formed on a right image receiving device.
FIG. 4C is a schematic diagram of an image processing by an image processing circuit in which a front lens illustrated in FIG. 1 is inserted between a tested eye and an objective lens, in particular, a schematic diagram of a writing processing of both tested eye images onto a frame memory.
FIG. 4D is a schematic diagram of an image processing by an image processing circuit in which a front lens illustrated in FIG. 1 is inserted between a tested eye and an objective lens, in particular, a schematic diagram of a read out processing of a tested eye composite image written onto the frame memory.
FIG. 4E is a schematic diagram of an image processing by an image processing circuit in which a front lens illustrated in FIG. 1 is inserted between a tested eye and an objective lens, in particular, a diagram that illustrates a state of display of the a tested eye composite image displayed on a monitoring device illustrated in FIG. 1.
FIG. 5 is a schematic diagram that illustrates a constitution of an optical system of a stereo video microscope apparatus according to a second embodiment of the present invention.
FIG. 6A is a schematic diagram of an image processing by an image processing circuit in which a front lens illustrated in FIG. 5 is not inserted between a tested eye and an objective lens, in particular, a left tested eye image formed on a left image receiving device.
FIG. 6B is a schematic diagram of an image processing by an image processing circuit in which a front lens illustrated in FIG. 5 is not inserted between a tested eye and an objective lens, in particular, a right tested eye image formed on a right image receiving device.
FIG. 6C is a schematic diagram of an image processing by an image processing circuit in which a front lens illustrated in FIG. 5 is not inserted between a tested eye and an objective lens, in particular, a schematic diagram of a writing processing of both tested eye images onto a frame memory.
FIG. 6D is a schematic diagram of an image processing by an image processing circuit in which a front lens illustrated in FIG. 5 is not inserted between a tested eye and an objective lens, in particular, a schematic diagram of a read out processing of a tested eye composite image written onto the frame memory.
FIG. 6E is a schematic diagram of an image processing by an image processing circuit in which a front lens illustrated in FIG. 5 is not inserted between a tested eye and an objective lens, in particular, a diagram that illustrates a state of display of the a tested eye composite image displayed on a monitoring device illustrated in FIG. 1.
FIG. 7A is a schematic diagram of an image processing by an image processing circuit in which a front lens illustrated in FIG. 5 is inserted between a tested eye and an objective lens, in particular, a right tested eye image formed on a left image receiving device.
FIG. 7B is a schematic diagram of an image processing by an image processing circuit in which a front lens illustrated in FIG. 5 is inserted between a tested eye and an objective lens, in particular, a left tested eye image formed on a right image receiving device.
FIG. 7C is a schematic diagram of an image processing by an image processing circuit in which a front lens illustrated in FIG. 5 is inserted between a tested eye and an objective lens, in particular, a schematic diagram of a writing processing of both tested eye images onto a frame memory.
FIG. 7D is a schematic diagram of an image processing by an image processing circuit in which a front lens illustrated in FIG. 5 is inserted between a tested eye and an objective lens, in particular, a schematic diagram of a read out processing of a tested eye composite image written onto the frame memory.
FIG. 7E is a schematic diagram of an image processing by an image processing circuit in which a front lens illustrated in FIG. 5 is inserted between a tested eye and an objective lens, in particular, a diagram that illustrates a state of display of the a tested eye composite image displayed on a monitoring device illustrated in FIG. 1.

### [Embodiment 1]

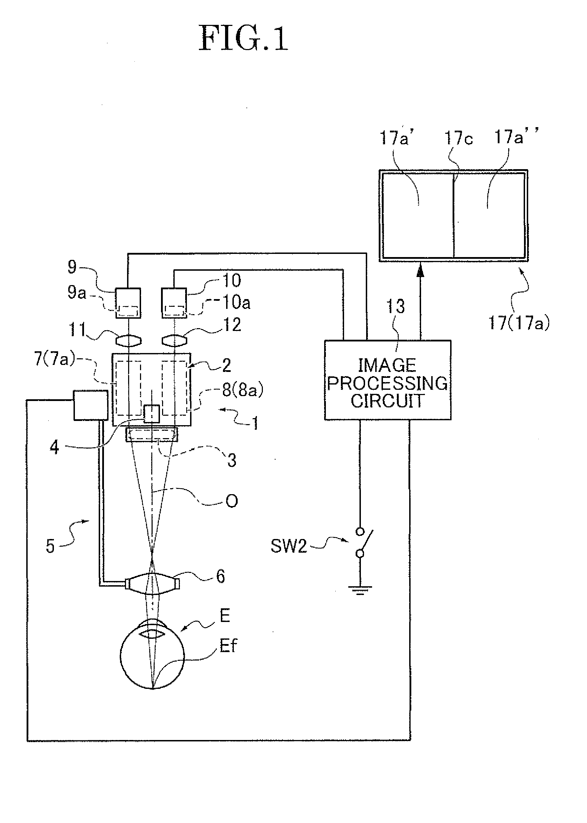

FIG. 1 is an approximate constitutional diagram that illustrates an embodiment 1 of a binocular stereo video microscope apparatus according to the present invention.

In FIG. 1, reference numeral E is a tested eye, reference numeral 1 illustrates a binocular stereo microscope apparatus. The binocular stereo microscope apparatus 1 includes a microscope lens barrel 2. An objective lens 3 and an illumination unit 4 are disposed in the microscope lens barrel 2. The illumination unit 4 (illumination optical system) is used to illuminate an interior of the tested eye E. A holding arm mechanism 5 is disposed in the microscope lens barrel 2. A front lens 6 is held in a leading end of the holding arm mechanism 5. The front lens 6 can be inserted between a tested eye E and an objective lens 3. The front lens 6 can also be detached thereof and is used for observing the interior of the tested eye E.

A pair of observatory optical systems 7 and 8 is disposed within the microscope lens barrel 2 at a right and left symmetrical position with an optical axis O of the objective lens 3 set as a boundary. The pair of observatory optical systems 7 and 8 is hereby approximately constituted from variable magnification lens systems 7a and 8a. A left TV camera 9 is disposed in the left variable magnification lens system 7a. A right TV camera 10 is disposed in the right variable magnification lens system 8a. The left TV camera 9 includes a left image receiving device 9a. The right TV camera 10 includes a right image receiving device 10a.

An imaging lens 11 is disposed between the left variable magnification lens system 7a thereof and the left TV camera 9. An imaging lens 12 is disposed between the right variable magnification lens system 8a thereof and the right TV camera 10. The imaging lens 11 forms a tested eye image on the left image receiving device 9a based on image forming light beams guided to the left observatory optical system 7. The imaging lens 12 forms a tested eye image on the right image receiving device 10a based on image forming light beams guided to the right observatory optical system 8.

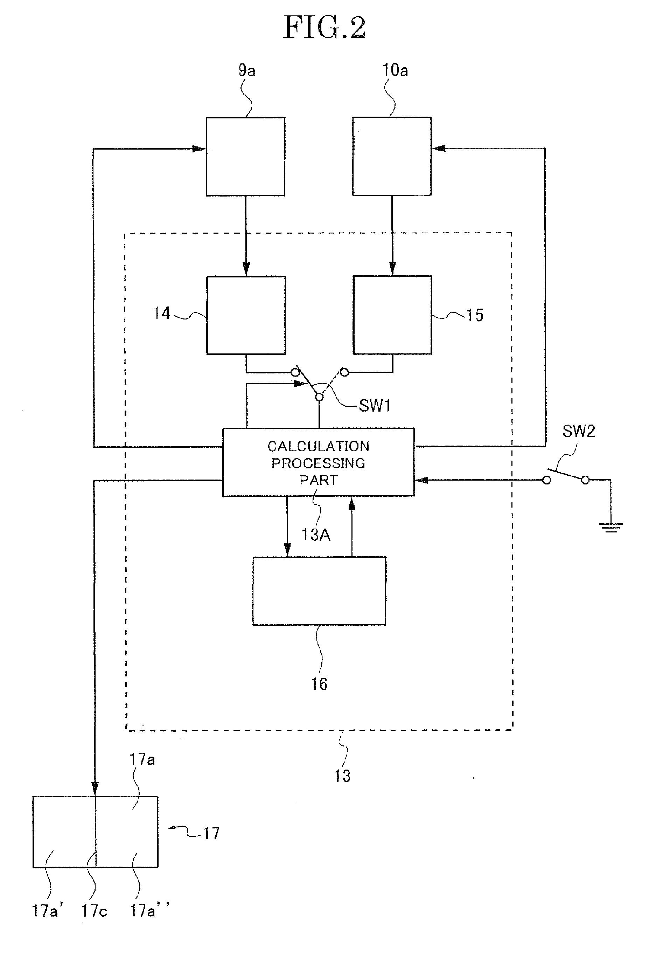

An image processing circuit 13 is connected to the TV camera 9 and 10. The image processing circuit 13, as illustrated in FIG. 2, includes a calculation processing circuit part 13A, a left buffer memory 14, a right buffer memory 15, a frame memory 16, a switch-over switch SW1, an inverted image cancellation switch SW2. The image processing circuit 13 is connected to a monitoring device 17. Reference numeral 17a is a display screen of the monitoring device 17. The display screen is divided into a left screen part 17a' and a right screen part 17a" with a center thereof set as a boundary. A light shielding plate 17c is disposed at a central position of the screen to be orthogonal against the screen 17a so that a tested eye image displayed on a left screen part 17a' is only observable by a left eye and a tested eye image displayed on a right screen part 17a" is only observable by a right eye.

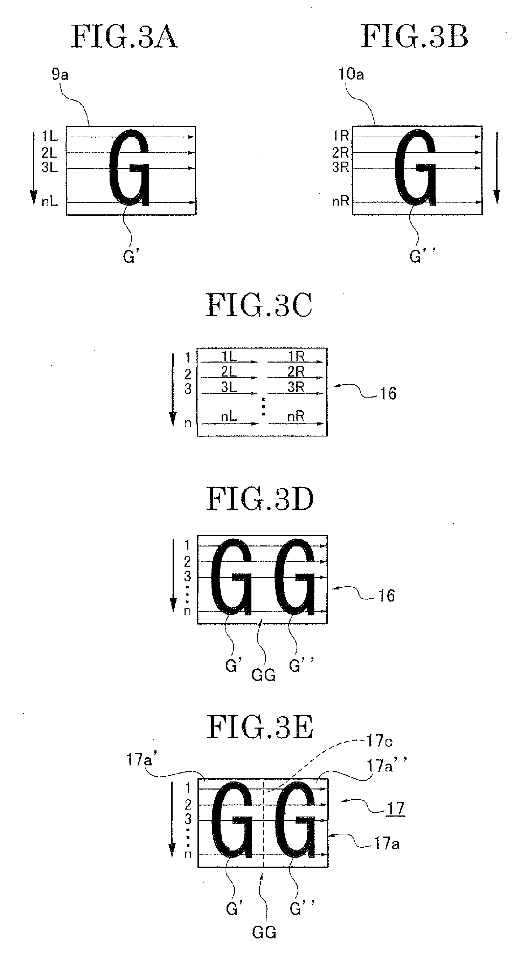

The observatory optical systems 7 and 8 hereby have an optical constitution in which image forming light beams experience an even number of times (including 0) of reflections to be guided to TV cameras 9 and 10 thereafter so that tested eye images are formed on both image receiving devices 9a and 10a. When the front lens 6 is not inserted between the tested eye E and the objective lens 3, as illustrated in FIG. 3A, a tested eye image G' to be formed on the left image receiving device 9a disposed in the left observatory optical system 7 is formed as a regular image on the left image receiving device 9a and a tested eye image G" to be formed on the right image receiving device 10a disposed in the right observatory system 8 is formed as a regular image on the right image receiving device 10a.

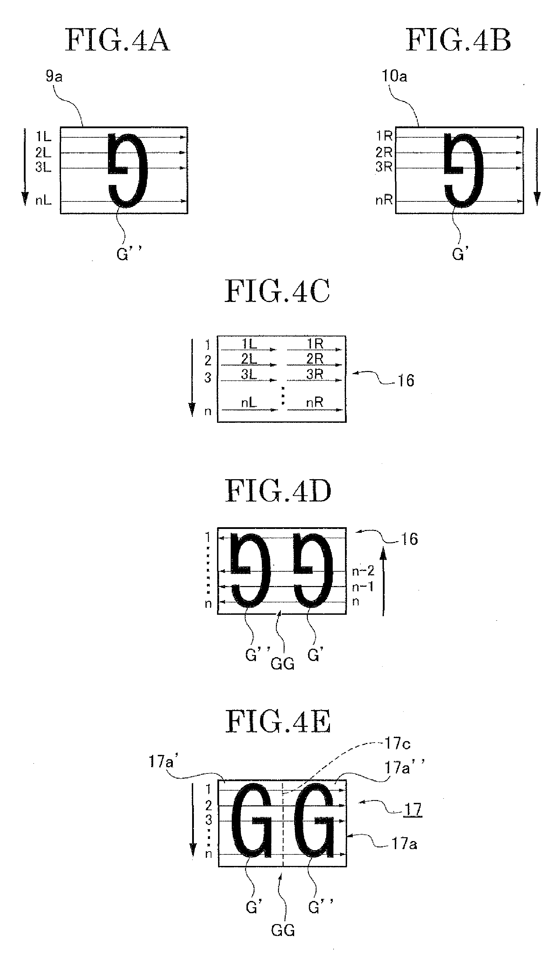

In addition, when the front lens 6 is inserted between the tested eye E and the objective lens 3, as illustrated in FIG. 4a, the tested eye image G" to be formed on the right image receiving device 10a disposed in the right observatory optical system 8 when the front lens 6 is not inserted between the tested eye E and the objective lens 3 is actually formed on the left image receiving device 9a as an inverted image in which the image is inverted in up and down as well as right and left directions (an image obtained by rotating 180 degrees the regular image around the screen center). In addition, as illustrated in FIG. 4B, a tested eye image G' to be formed on the left image receiving device 9a disposed in the left observatory optical system 7 when the front lens 6 is not inserted between the tested eye E and the objective lens 3 is actually formed on the right image receiving device 10a as an inverted image in which the image is inverted in up and down as well as right and left directions.

The image processing circuit 13 performs a processing described hereinbelow.

### (1) when the front lens 6 is not inserted between the tested eye E and the objective lens 3

In this case, as described using FIG. 3A and FIG. 3B, a tested eye image G' to be formed on the left image receiving device 9a disposed in the left observatory optical system 7 is formed on the left image receiving device 9a as a regular image (upright image). As illustrated in FIG. 3B, a tested eye image G" to be formed on the right image receiving device 10a disposed in the right observatory optical system 8 is formed on the right image receiving device 10a as a regular image (upright image).

Hereby the fact that both tested eye images G' and G" are regular images means tested eye images formed on both image receiving devices 9a and 10a are either an upright image or an inverted image because whether tested eye images G' and G" are formed as upright images against both image receiving devices 9a and 10a or formed as inverted images depends on lens constitutions of the observatory optical system 7 and 8 as well as disposed postures of the TV camera 9 and 10.

The left buffer memory 14 is used for storing temporarily pixel data read out from each pixel of the left image receiving device 9a. The right buffer memory 15 is used for storing temporarily pixel data read out from each pixel of the right image receiving device 10a. The frame memory 16 is used for storing a composite image GG obtained by synthesizing both tested eye images G' and G".

The left buffer memory 14 and the right buffer memory 15 are switched over alternatively by a switch over switch SW1 whenever a single line of pixel data is read out.

The calculation processing part 13A, as illustrated in FIG. 3A. and 3B, reads out pixel data from each pixel from an upper left corner towards a lower right corner. That is, first, each pixel of an uppermost line 1L of the left image receiving device 9a is scanned from left towards right. Next, each pixel of lines 2L, 3L, ... , nL is scanned sequentially from left to right towards a bottom of the left image receiving device 9a.

Therefore, pixel data of pixels from the read out line 1L to the read out line nL is stored in the left buffer memory 14.

In the same way, the calculation processing part 13A scans from left towards right each pixel of an uppermost line 1R of the right image receiving device 10a. Next, each pixel of lines 2R through nR is scanned sequentially from left to right towards a bottom of the right image receiving device 10a. Therefore, pixel data of pixels from the read out line 1R to the read out line nR is stored in the right buffer memory 15.

Next, the calculation processing part 13A scans the left buffer memory 14, in particular, a line 1L of the left buffer memory 14 from left towards right and reads out pixel data of line 1L. The calculation processing part 13A, at a timing in which pixel data of line 1L is read out, switches over a switch-over switch SW1 so that the switch-over switch SW1 is switched over from a state connected to the left buffer memory 14 (illustrated in solid lines in FIG. 2) to a state connected to the right buffer memory 15 (illustrated in broken lines in FIG. 2). Next, the calculation processing part 13A scans the right buffer memory 15, in particular, a line 1R of the right buffer memory 15 from left towards right and reads out pixel data of line 1R. As illustrated in FIG. 3C, pixel data of line 1L and line 1R read out in such a way is written by the calculation processing part 13A onto each pixel memory of line 1 of the frame memory 16.

Next, the calculation processing part 13A, at a timing in which pixel data of line 1R is read out, switches over a switch-over switch SW1 so that the switch-over switch SW1 is switched over from a state connected to the right buffer memory 15 to a state connected to the left buffer memory 14. Next, the calculation processing part 13A scans the left buffer memory 14, in particular, a line 2L of the left buffer memory 14 from left towards right and reads out pixel data of line 2L.

The calculation processing part 13A, at a timing in which pixel data of line 2L is read out, then switches over a switch-over switch SW1 so that the switch-over switch SW1 is switched over from a state connected to the left buffer memory 14 to a state connected to the right buffer memory 15. Next, the calculation processing part 13A scans the right buffer memory 15, in particular, a line 2R of the right buffer memory 15 from left towards right and reads out pixel data of line 2R. Pixel data of line 2L and line 2R read out in such a way is written by the calculation processing part 13A onto each pixel memory of line 2 of the frame memory 16.

In such a way, read out of a single line of pixel data from both buffer memories 14 and 15 is repeated alternatively by the calculation processing part 13A. The calculation processing part also repeats writing of the read out pixel data onto the frame memory 16. Both processes are repeated until all pixel data of the left buffer memory 14 and all pixel data of the right buffer memory 15 are written onto each pixel memory of n number of lines of the frame memory 16.

By the consecutive read out processing and writing processing of the calculation processing part 13A, the tested eye images G' and G" formed on separate image receiving devices 9a and 10a is synthesized to a single frame memory 16 and stored as a tested eye composite image GG.

For example, as illustrated in a frame format in FIG. 3A and FIG. 3B, in the case an image of one letter "G" is formed as an upright image on separate image receiving devices 9a and 10a, two parallel letters of "G" are stored and recorded as an upright image as illustrated in FIG. 3D.

The calculation processing part 13A, after implementing these consecutive processings, reads out pixel data of the frame memory 16 from an upper left corner to a lower right corner with regard to each pixel of the frame memory 16. That is, the calculation processing part 13A, as illustrated in FIG. 3D, scans line 1 from left towards right and reads out pixel data of line 1L. Next, the image processing circuit scans towards the bottom and from left towards right pixel memories of line 2, line 3, ... , line n and reads out pixel data of line 2, line 3, ... , line n. In such a way, the calculation processing circuit 13A, as illustrated in FIG. 3E, forms based on pixel data dot images on a screen 17a of the monitoring device 17 in sequence from an upper left corner towards a lower right corner following a sequence of the read out pixel data.

In such a way, on the screen 17a of the monitoring device 17, within two letters of "G", the tested eye image G' to be displayed in the left is displayed in the left screen part 17a' and the tested eye image G" to be displayed in the right is displayed in the right screen part 17a". Both images are together disposed in parallel as a regular image "G". The tester simultaneously views the left tested eye image G' by only the left eye and the right tested eye image G" by only the right eye so that the tested eye composite image GG can be observed sterically.

That is, a concave point of an ocular fundus of the tested eye can be viewed as concave by the tester. A convex point can be viewed as convex by the tester. As a result, erroneous judgments can be avoided. (2) when the front lens 6 is inserted between the tested eye E and the objective lens 3
In this case, as just described in FIG. 4A and FIG. 4B, the tested eye image G" to be formed on the right image receiving device 10a disposed in the right observatory optical system 8 when the front lens 6 is not inserted between the tested eye E and the objective lens 3, is actually formed on the left image receiving device 9a as an inverted image inverted in up and down as well as right and left directions. As illustrated in FIG. 4B, the tested eye image G' to be formed on the left image receiving device 9a disposed in the left observatory optical system 7 when the front lens 6 is not inserted between the tested eye E and the objective lens 3, is actually formed on the right image receiving device 10a as an inverted image inverted in up and down as well as right and left directions.

Image processings by the calculation processing part 13A from when pixel data is read out from each image receiving devices 9a and 10a to when tested eye composite images GG is synthesized to be stored in the frame memory 16 are the same to image processings when the front lens 6 is not inserted between the tested eye E and the objective lens 3 (refer to FIG. 4A through FIG. 4C).

The calculation processing part 13A, when the inverted image cancellation switch is manipulated to be turned on or when the inverted image cancellation switch SW2 is automatically turned on due to insertion of the front lens 6 between the tested eye E and the objective lens 3, scan each pixel memory of the frame memory 16 from a lower right corner to an upper left corner. That is, as illustrated in FIG. 4D, the calculation processing part 13A reads out from right towards left of line n pixel data stored in pixel memory. Next, the calculation processing part 13A reads out pixel data from right towards left of line n-1. This is repeated until read out of pixel data of line 1 is finished. The calculation processing part 13A, as illustrated in FIG. 4E, forms based on pixel data dot images on a screen 17a of the monitoring device 17 in sequence from an upper left corner towards a lower right corner following a sequence of the read out pixel data. Based on these pixel data, the tested eye composite image GG is displayed on the screen 17a of the monitoring device 17.

In such a way, on the screen 17a of the monitoring device 17, inverted images formed to be inverted in up and down as well as right and left directions are displayed as tested eye images G' and G" of a regular state on both image receiving devices 9a and 10a in the same way to when the front lens 6 is not inserted between the tested eye E and the objective lens 3.

For example, as illustrated in FIG. 4A and FIG. 4B in a frame format, in the case an image of one letter "G" is formed to be inverted in up and down as well as right and left directions on separate image receiving devices 9a and 10a, two parallel letters "G" are recorded and stored in the frame memory 16 in a state inverted in up and down as well as right and left directions. A processing is performed in which pixel data of the letter "G" recorded and stored in the frame memory 16 and inverted in up and down as well as right and left directions is read out in a reverse sequence and displayed on a screen 17a of the monitoring device 17. Tested eye images G' and G" formed on both image receiving devices 9a and 10a and inverted in up and down as well as right and left directions is displayed as a tested eye composite image GG on the screen 17a of the monitoring device 7 in a state the same to when the front lens 6 is not inserted between the tested eye E and the objective lens 3.

The tester simultaneously views the left tested eye image G' by only the left eye and the right tested eye image G" by only the right eye so that the tested eye composite image GG can be observed sterically.

### (Embodiment 2)

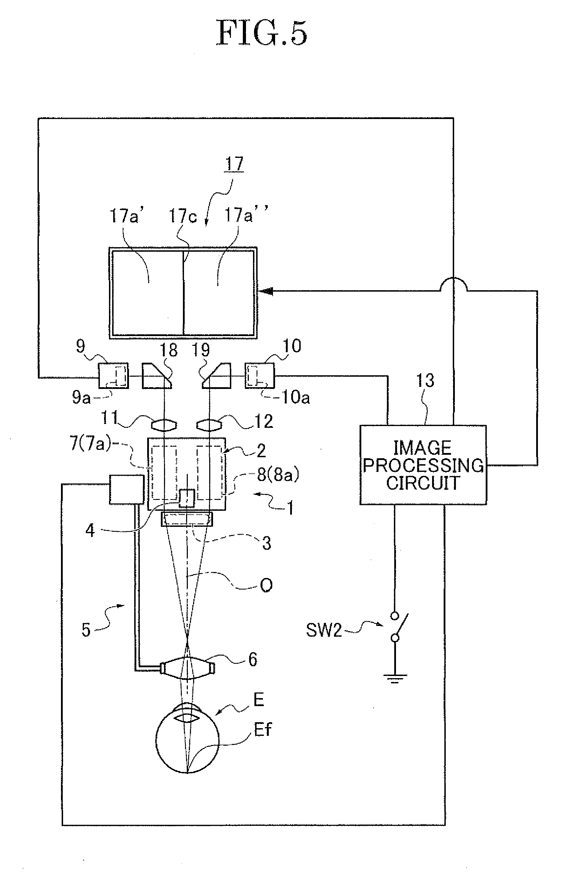

FIG. 5 is an approximate constitutional diagram illustrating a second embodiment of a binocular stereo video microscope apparatus according to the present invention.

In the FIG. 5, 18 is a left reflective prism, 19 is a right reflective prism. The left reflective prism is disposed between the imaging lens 11 and the left TV camera 9. The right reflective prism 19 is disposed between the imaging lens 11 and the right TV camera 10.

The binocular stereo video microscope apparatus illustrated in FIG. 5 is a representative example in which image forming light beams guided to observatory optical systems 7, 8 receive an odd number of times of reflections to be formed on both image receiving devices 9a and 10a. The remaining constitutions are approximately the same to the first embodiment so that detailed descriptions of the same constituent elements are abbreviated and only differing parts are described.

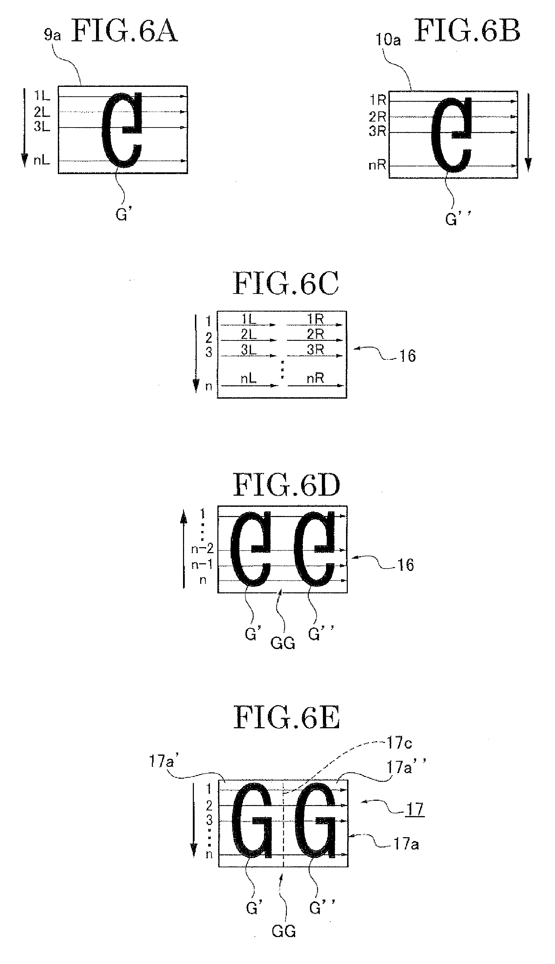

According to the second embodiment, when the front lens 6 is not inserted between the tested eye E and the objective lens 3, as illustrated in FIG. 6A, a tested eye image G' to be formed on the left image receiving device 9a disposed in the left observatory optical system 7 is actually formed on the left image receiving device 9a as an image flipped over in up and down directions. As illustrated in FIG. 6B, a tested eye image G" to be formed on the right image receiving device 10a disposed in the right observatory optical system 8 is actually formed on the right image receiving device 10a as an image flipped over in up and down directions.

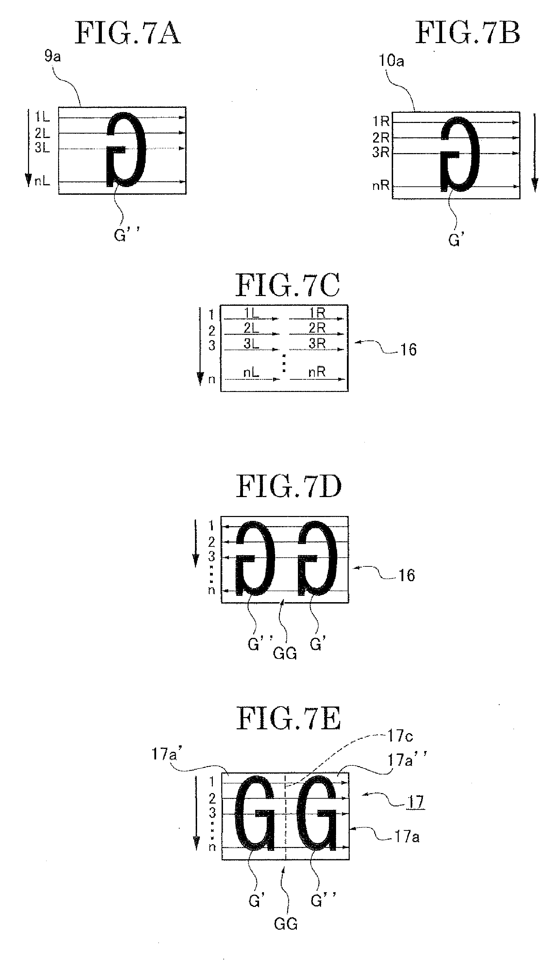

In addition, when the front lens 6 is inserted between the tested eye E and the objective lens 3, as illustrated in FIG. 7A, the tested eye image G" to be formed on the right image receiving device 10a disposed in the right observatory optical system 8 when the front lens 6 is not inserted between the tested eye E and the objective lens 3, is actually formed on the left image receiving device 9a as an inverted image inverted in up and down as well as right and left directions. In addition, as illustrated in FIG. 7B, the tested eye image G' to be formed on the left image receiving device 9a disposed in the left observatory optical system 7 when the front lens 6 is not inserted between the tested eye E and the objective lens 3, is actually formed on the right image receiving device 10a as an inverted image inverted in up and down as well as right and left directions.

Put it in another way, in the second embodiment, in the case the front lens 6 is inserted between the tested eye E and the objective lens 3, a tested eye image G" is formed on the left image receiving device 9a as illustrated in FIG. 7A to be flipped over in up and down directions against the tested eye image G" (refer to FIG. 4A) formed on the left image receiving device 9a when the front lens of the first embodiment is inserted between the tested eye E and the objective lens 3. In addition, in the second embodiment, in the case the front lens 6 is inserted between the tested eye E and the objective lens 3, a tested eye image G' is formed on the right image receiving device 10a as illustrated in FIG. 7B to be flipped over in up and down directions against the tested eye image G' (refer to FIG. 4B) formed on the right image receiving device 10a when the front lens of the first embodiment is inserted between the tested eye E and the objective lens 3.

The calculation processing circuit 13 performs the processings described hereinbelow.

### (1) when the front lens 6 is not inserted between the tested eye E and the objective lens 3

In this case, as described using FIG. 6A and FIG. 6B, the tested eye image G' to be formed on the left image receiving device 9a disposed in the left observatory optical system 7 is actually formed on the left image receiving device 9a as an image flipped over in up and down directions. As illustrated in FIG. 6B, the tested eye image G" to be formed on the right image receiving device 10a disposed in the right observatory optical system 8 is actually formed on the right image receiving device 10a as an image flipped over in up and down directions.

From when pixel data is read out from each image receiving devices 9a and 10a to when the tested eye composite image GG is synthesized to be stored in the frame memory 16, image processings by the calculation processing part 13A is the same to image processings of FIG. 3A through 3C or FIG. 4A through 4C of the first embodiment so that descriptions of which are hereby abbreviated.

Based on image processings by the calculation processing part 13A, as illustrated in FIG. 6D, a tested eye composite image GG flipped over in up and down directions are formed on the frame memory 16.

The calculation processing part 13A, after implementing the consecutive processings, reads out pixel data of the frame memory 16 from a lower left corner towards an upper right corner with regard to each pixel of the frame memory 16. That is, the calculation processing part 13A scans from left to right of line n and reads out pixel data of line nL. Next, the calculation processing part 13A scans from left towards right pixel memories of line n-1, line n-2, ... , line 1 towards a top so that pixel data of line n-1, line n-2, ... , line 1 is read out. In such a way, the calculation processing circuit 13A, as illustrated in FIG. 6E, forms based on pixel data dot images on a screen 17a of the monitoring device 17 in sequence from an upper left corner towards a lower right corner following a sequence of the read out pixel data.

In such a way, on the screen 17a of the monitoring device 17, within two letters of "G", the tested eye image G' to be displayed in the left is displayed in the left screen part 17a' and the tested eye image G" to be displayed in the right is displayed in the right screen part 17a". Both images are together disposed in parallel as a regular image "G". The tester simultaneously views the left tested eye image G' by only the left eye and the right tested eye image G" by only the right eye so that the tested eye composite image GG can be observed sterically.

### (2) when the front lens 6 is inserted between the tested eye E and the objective lens 3

In this case, as described in FIG. 7A and FIG. 7B, the tested eye image G" to be formed on the right image receiving device 10a disposed in the right observatory optical system 8 when the front lens 6 is not inserted between the tested eye E and the objective lens 3, is actually formed on the left image receiving device 9a as an inverted image inverted in up and down as well as right and left directions. In addition, as illustrated in FIG. 7B, the tested eye image G' to be formed on the left image receiving device 9a disposed in the left observatory optical system 7 when the front lens 6 is not inserted between the tested eye E and the objective lens 3, is actually formed on the right image receiving device 10a as an inverted image inverted in up and down as well as right and left directions.

From when pixel data is read out from each image receiving devices 9a and 10a to when the tested eye composite image GG is synthesized to be stored in the frame memory 16, image processings by the calculation processing part 13A is the same to image processings of when the front lens 6 is not inserted between the tested eye E and the objective lens 3 (refer to FIG. 7A through FIG. 7C).

The calculation processing part 13A, when the inverted image cancellation switch is manipulated to be turned on or when the inverted image cancellation switch SW2 is automatically turned on due to insertion of the front lens 6 between the tested eye E and the objective lens 3, scan each pixel memory of the frame memory 16 from an upper right corner to a lower left corner. That is, as illustrated in FIG. 7D, the calculation processing part 13A reads out from right towards left of line 1 pixel data stored in pixel memory. Next, the calculation processing part 13A reads out pixel data from right towards left of line 2. This is repeated until read out of pixel data of line n is finished. The calculation processing part 13A, as illustrated in FIG. 7E, forms based on pixel data dot images on a screen 17a of the monitoring device 17 in sequence from an upper left corner towards a lower right corner following a sequence of the read out pixel data. Based on these pixel data, the tested eye composite image GG is displayed on the screen 17a of the monitoring device 17.

In such a way, on the screen 17a of the monitoring device 17, tested eye images G' and G" formed on both image receiving devices 9a and 10a to be inverted in up and down as well as right and left directions are displayed as a tested eye composite images GG of a same state to when the front lens 6 is not inserted between the tested eye E and the objective lens 3 in which an optical system illustrated in FIG. 1 is used for observation.

According to the present invention, a binocular stereo video microscope apparatus can be provided in which image forming relationships can return to a normal state even if an inverter device constituted from an optical device that inverts image forming relationships is not used, and stereo image can be produced for a tester.

## Claims

1. A binocular stereo video microscope apparatus, comprising:
an illumination optical system (4) that illuminates a tested eye (E),
a right and left pair of observatory optical system (7, 8) that receives via an objective lens (3) image forming light beams from a tested eye (E) and forms an image of the tested eye (E) respectively on both image receiving devices (9a, 10a) of a right and left pair,
a monitoring device (17) that produces each tested eye image on a same screen but sorting one tested eye image to the left and sorting another tested eye image to the right with a center set as a boundary thereof in which the tested eye images are obtained via the right and left pair of the observatory optical system (7, 8),
a front lens (6) inserted between the tested eye (E) and the objective lens (3) for observing an interior of the tested eye (E),
an inverted image cancellation switch (SW2) that cancels an inverted relationship of the tested eye images inverted in up and down as well as right and left directions against the tested eye (E) in which the tested eye images are formed on each image receiving devices (9a, 10a) via the right and left pair of observatory optical system (7, 8) based on the insertion of the front lens (6) between the tested eye (E) and the objective lens (3), and
an image processing circuit (13) that generates a composite image of the tested eye (E) based on pixel data obtained from both image receiving devices (9a, 10a) of the right and left pair, wherein
the image processing circuit (13) performs composite processing to the tested eye image formed on the left image receiving device (9a) and the tested eye image formed on the right image receiving device (10a) in which the both images keep a relationship as they are and are processed as a tested eye composite image regardless of whether the front lens (6) is inserted or not between the tested eye (E) and the objective lens (3),
based on a switch state of the inverted image cancellation switch (SW2), a read out processing and scanning of the a tested eye composite image are made different by the image processing circuit between when the front lens (6) is not inserted between the tested eye (E) and the objective lens (3) and when the front lens (6) is inserted between the tested eye (E) and the objective lens (3),
the image processing circuit implements a processing in which a tested eye image based on image forming light beams to be obtained via a left observatory optical system (7) in a left part of the same screen when the front lens (6) is not inserted between the tested eye (E) and the objective lens (3) is produced as a regular image corresponding to the tested eye (E), and, a tested eye image based on image forming light beams to be obtained via a right observatory optical system (8) in a right part of the same screen when the front lens (6) is not inserted between the tested eye (E) and the objective lens (3) is produced as a regular image corresponding to the tested eye (E).

2. The binocular stereo video microscope apparatus according to Claim 1, wherein
the right and left pair of optical system (7, 8) adopts an optical constitution in which in the case the front lens (6) is not inserted between the tested eye (E) and the objective lens (3), a tested eye image based on image forming light beams obtained through the left observatory optical system (7) is formed on the left image receiving device (9a) as a regular image, a tested eye image based on image forming light beams obtained through the right observatory optical system (8) is formed on the right image receiving device (10a) as a regular image, and
another optical constitution is adopted in the case the front lens (6) is inserted between the tested eye (E) and the objective lens (3) in which a tested eye image based on image forming light beams to be obtained through the right observatory optical system (8) when the front lens (6) is not inserted between the tested eye (E) and the objective lens (3), is actually formed on the left image receiving device (9a) as an inverted image inverted in up and down as well as right and left directions against the regular image, and, a tested eye image based on image forming light beams to be obtained through the left observatory optical system (7) when the front lens (6) is not inserted between the tested eye (E) and the objective lens (3), is actually formed on the right image receiving device (10a) as an inverted image inverted in up and down as well as right and left directions against the regular image.

3. The binocular stereo video microscope apparatus according to Claim 2, wherein
in the case the front lens (6) is not inserted between the tested eye (E) and the objective lens (3), the image processing circuit implements a processing that synthesizes both of the regular images to produce a tested eye image obtained through the left image receiving device on a left part of the same screen and a tested eye image obtained through the right image receiving device on a right part of the same screen, and
in the case the front lens is inserted between the tested eye and the objective lens, the image processing circuit (13) implements a processing that synthesizes both of the images inverted in up and down as well as right and left directions as they are to create a composite image inverted in up and down as well as right and left directions in which in order to cancel based on a switch state of the inverted image processing switch (SW2) up and down inversions of the images inverted in up and down as well as right and left directions, a tested eye image of the right image receiving device (10a) based on image forming light beams to be obtained through the left observatory optical system (7) when the front lens (6) is not inserted between the tested eye (E) and the objective lens (3) is actually produced on a left part of the same screen and a tested eye image of the left image receiving device (9a) based on image forming light beams to be obtained through the right observatory optical system (8) when the front lens (6) is not inserted between the tested eye (E) and the objective lens (3) is actually produced on a right part of the same screen.

4. The binocular stereo video microscope apparatus according to Claim 1, wherein
the right and left pair of optical system (7, 8) adopts an optical constitution in which in the case the front lens (6) is not inserted between the tested eye (E) and the objective lens (3), a tested eye image based on image forming light beams obtained through the left observatory optical system (7) is formed on the left image receiving device (9a) as an image inverted in up and down directions, a tested eye image based on image forming light beams obtained through the right observatory optical system (8) is formed on the right image receiving device (10a) as an image inverted in up and down directions, and
another optical constitution is adopted in the case the front lens (6) is inserted between the tested eye (E) and the objective lens (3) in which a tested eye image based on image forming light beams to be obtained through the right observatory optical system (8) when the front lens (6) is not inserted between the tested eye (E) and the objective lens (3), is actually formed on the left image receiving device (9a) as an image inverted in up and down as well as right and left directions against the inverted image inverted in up and down directions, and, a tested eye image based on image forming light beams to be obtained through the left observatory optical system (7) when the front lens (6) is not inserted between the tested eye (E) and the objective lens (3), is actually formed on the right image receiving device (10a) as an image inverted in up and down as well as right and left directions against the inverted image inverted in up and down directions.

5. The binocular stereo video microscope apparatus according to Claim 4, wherein
in the case the front lens (6) is not inserted between the tested eye (E) and the objective lens (3), the image processing circuit (13) implements a read out processing and scanning of a tested eye composite image formed by synthesizing both inverted images inverted in up and down directions in which the inverted images inverted in up and down directions are displayed on the same screen as regular images, and, a tested eye image to be obtained through the left image receiving device (9a) is produced on a left part of the same screen, and, a tested eye image to be obtained through the right image receiving device (10a) is produced on a right part of the same screen,
in the case the front lens (6) is inserted between the tested eye (E) and the objective lens (3), the image processing circuit (13) implements an image processing in which up and down inversions of the composite image inverted in up and down as well as right and left directions are cancelled based on a switch state of the inverted image processing switch (SW2), and, a tested eye image of the right image receiving device (10a) based on image forming light beams to be obtained through the left observatory optical system (7) when the front lens (6) is not inserted between the tested eye (E) and the objective lens (3) is actually produced on a left part of the same screen, and, a tested eye image of the left image receiving device (9a) based on image forming light beams to be obtained through the right observatory optical system (8) when the front lens (6) is not inserted between the tested eye (E) and the objective lens (3) is actually produced on a right part of the same screen.

6. The binocular stereo video microscope apparatus according to Claim 1, wherein
the inverted image cancellation switch (SW2) is turned on and off by manual operation.

7. The binocular stereo video microscope apparatus according to Claim 1, wherein
the inverted image cancellation switch (SW2) is interlocked to the insertion of the front lens (6) into a light path between the tested eye (E) and the objective lens (3) to be turned on and off.
